(19) Europäisches Patentamt
European Patent Office
Office européen des brevets

(11) EP 4 177 241 A1

(12) **EUROPEAN PATENT APPLICATION**
published in accordance with Art. 153(4) EPC

(43) Date of publication:
**10.05.2023 Bulletin 2023/19**

(21) Application number: **20943432.3**

(22) Date of filing: **26.10.2020**

(51) International Patent Classification (IPC):
***C07C 59/125*** *(2006.01)* ***C07C 51/367*** *(2006.01)*
***C22B 3/12*** *(2006.01)* ***C22B 23/00*** *(2006.01)*
***C22B 47/00*** *(2006.01)*

(52) Cooperative Patent Classification (CPC):
Y02P 10/20; Y02W 30/84

(86) International application number:
**PCT/CN2020/123531**

(87) International publication number:
**WO 2022/000881 (06.01.2022 Gazette 2022/01)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.06.2020 CN 202010599602**

(71) Applicant: **Botree Cycling Sci & Tech Co., Ltd.**
**Beijing 100083 (CN)**

(72) Inventor: **WANG, Xue**
**Beijing 100083 (CN)**

(74) Representative: **Cesa, Roberta**
**Buzzi, Notaro & Antonielli d'Oulx S.p.A.**
**Corso Vittorio Emanuele II, 6**
**10123 Torino (IT)**

# (54) CARBOXYLIC ACID COMPOUND, AND PREPARATION METHOD THEREFOR AND APPLICATION THEREOF

(57) Provided are a carboxylic acid compound of formula I, and a preparation method therefor and application thereof. When being applied to the extraction and separation of metal ions, the carboxylic acid compound can achieve a high separation coefficient, low back extraction acidity, high load rate, high back extraction rate, high stability, and low water solubility, so that the extraction process is stable, and environmental pollution and components can be reduced. The present application can be used in various systems such as ternary battery recycling and battery-grade nickel sulfate preparation.

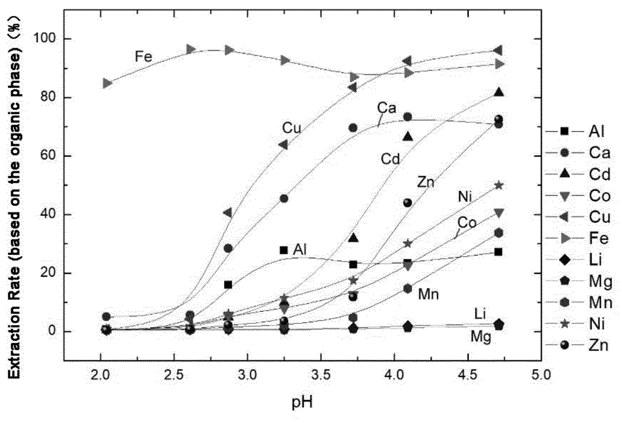


FIG. 1



Processed by Luminess, 75001 PARIS (FR)

## Description

CROSS-REFERENCE TO RELATED APPLICATION(S)

This application claims priority to Chinese Patent Application No. 202010599602.9 filed Jun. 28, 2020, the disclosure of which is incorporated herein by reference in its entirety.

TECHNICAL FIELD

The present application relates to a carboxylic acid compound, a preparation method therefor and an application thereof.

BACKGROUND

In recent years, with the rapid development and popularization of electric vehicles, the demand scale of lithium-ion batteries is increasingly expanding. The nickel-cobalt-manganese ternary positive electrode material has good cycle performance, stable structure and high performance cost ratio, and is a new type of positive electrode material for lithium ion batteries. The main raw materials of precursor products of the ternary positive electrode material are nickel salt, cobalt salt and manganese salt.

Cobalt and nickel are usually generated together, and often occur together in ore, such as nickel laterite ore. In many industries, there are waste residues containing valuable metals such as nickel and cobalt generated, such as waste power battery materials, nickel-cobalt-containing waste residue, waste catalysts, etc. Most of these waste residues also contain manganese in high content, which has high recycling value, and can be recycled to prepare nickel-cobalt-manganese ternary precursors.

Solvent extraction technology is an effective technology to separate and extract various metals from solution, which has many advantages, such as high separation efficiency, simple process and equipment, continuous operation, easy to realize automatic control, etc., and has been continuously concerned and developed by many researchers. With the urgency of environmental protection and resource recycling, higher requirements are put forward for the energy consumption, acid consumption, sewage discharge and productivity of the extraction system. Therefore, it is necessary to improve the extraction efficiency, separation performance and solubility of the extractant to meet the environmental and economic requirements.

Commonly used cation-exchange extractants, such as acid phosphoric acid extractants P204, P507, C272, neutral complexing extractant TBP, chelating extractant LiX84 and carboxylic acid extractants Versatic10, Versatic911, etc., have been widely used in the separation and purification of metal elements because of their good extraction and separation performance. CN109449523A discloses a comprehensive recovery method of waste lithium-ion batteries, which comprises: firstly, a feed solution is adjusted to a pH of 4.2-4.5, and extracted with P204 to obtain a P204 raffinate and a loaded organic phase, and the loaded organic phase is back-extracted with sulfuric acid to obtain manganese sulfate; the P204 raffinate is adjusted to a pH of 4.5-5, and extracted with C272 to obtain a C272 raffinate and a load organic phase, and the C272 loaded organic phase is back-extracted with sulfuric acid to obtain cobalt sulfate solution; the C272 raffinate is adjusted to a pH of 5-5.5, and extracted with P507 to obtain Ni, and the P507 loaded organic phase is back-extracted with sulfuric acid to obtain nickel sulfate solution. However, these extractants have obvious shortcomings in the separation process: P507/P204 is used for the separation of nickel and cobalt, but it cannot simultaneously extract nickel, cobalt and manganese in the recovery of lithium-ion battery positive electrode materials; it has high cost to recovery nickel, cobalt and manganese separately, and the back extraction acidity is high, and the pollution is serious; C272 preferentially extracts calcium and magnesium before nickel, which has complicated operation procedures and high impurity removal cost; Versatic10 extractant has high solubility in the aqueous phase, which easily leads to process instability and environmental pollution.

SUMMARY

Aiming at the shortcomings of the prior art, the present application provides a carboxylic acid compound, a preparation method therefor and an application thereof. When used as an extractant, the carboxylic acid compound has good selectivity to ions (especially nickel, cobalt, manganese ions), low back extraction acidity, low water solubility, high stability and low cost.

The present application adopts the technical solutions below to solve the technical problems.

The present application provides a carboxylic acid compound shown in formula I or a salt thereof:

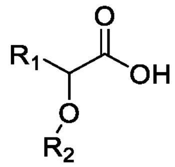

I ;

in formula I, $R_1$ and $R_2$ are independently selected from $C_3$-$C_{12}$ linear or branched alkyl.

In formula I, preferably, $R_1$ is $C_4$-$C_9$ linear or branched alkyl; more preferably, $R_1$ is $C_4$-$C_9$ linear alkyl, for example, n-butyl, n-pentyl, n-hexyl or n-octyl.

In formula I, preferably, $R_2$ is $C_3$-$C_{10}$ linear or branched alkyl; more preferably, $R_2$ is $C_6$-$C_8$ linear or branched alkyl; for example, $R_2$ is n-hexyl, n-octyl or isooctyl (for example,

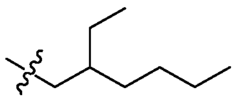 or 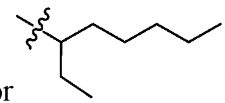 ).

In formula I, preferably, a total carbon number n of $R_1$ and $R_2$ is 10-20, and for example, n is 12, 14 or 16.

In formula I, preferably, the carboxylic acid compound shown in formula I is selected from any one of the following compounds:

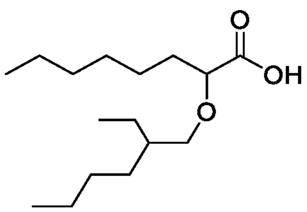 , 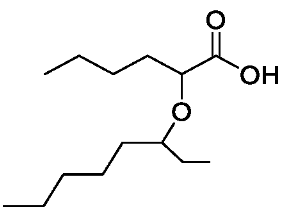 , 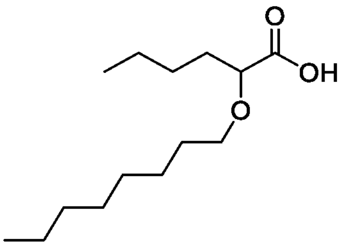 ,

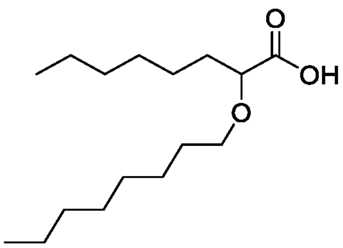 , 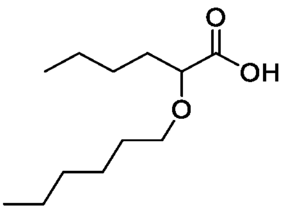 , or 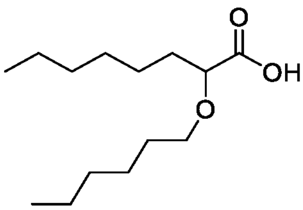 .

The salt of the carboxylic acid compound shown in formula I is generally prepared by reacting the carboxylic acid compound shown in the formula I with a base, for example, by reacting the carboxylic acid compound shown in the formula I with a base according to a molar ratio of 1:1.

The base can be a conventional base in the art, for example, alkali metal hydroxide or ammonia, such as sodium hydroxide, potassium hydroxide or ammonia; therefore, the salt of the carboxylic acid compound can be a sodium salt, a potassium salt or an ammonium salt.

A condition of the preparation method of the salt of the carboxylic acid compound shown in formula I can be the conventional conditions of acid-base salt-forming reaction in the art.

The carboxylic acid extractant shown in formula I can be extracted from natural substances or synthesized by conventional methods, and the extractant can be one or a mixture (for example, two or more) of the carboxylic acid shown in formula I when used for extraction.

The present application provides a preparation method for the carboxylic acid compound shown in formula I, which includes reacting a compound shown in formula II with a compound shown in formula III in a solvent under the action of a base;

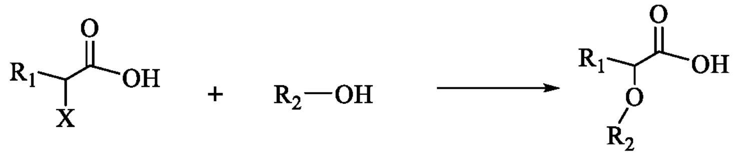

II III I

X is halogen, and R, and $R_2$ are as defined hereinabove.

In the preparation method, preferably, the halogen is fluorine, chlorine, bromine or iodine, for example, chlorine or bromine, or bromine.

In the preparation method, the solvent can be the solvent commonly used for such reaction in the art, for example, an ether solvent, and the ether solvent is, for example, tetrahydrofuran.

In the preparation method, an amount of the solvent can be the conventional amount of such reaction in the art, as long as the reaction is not affected. For example, the solvent and the compound shown in formula III have a volume-mass ratio of 1-10 mL/g, for example, 5.3 mL/g, 6.25 mL/g, 7.0 mL/g, 7.1 mL/g or 7.7 mL/g.

In the preparation method, the base can be the commonly used base in such reaction in the art, for example, an alkali metal or an alkali metal hydride, and for example, sodium or sodium hydride.

In the preparation method, an amount of the base can be the conventional amount of such reaction in the art, and for example, the base and the compound shown in formula II have a molar ratio of (1-1.5): 1, for example, 1.1:1, 1.2:1 or 1.35: 1.

In the preparation method, a molar ratio of the compound shown in formula II to the compound shown in formula III can be the conventional ratio of such reaction in the art, preferably 1:(1-1.5), for example, 1:1.1 or 1:1.2.

In the preparation method, a temperature of the reaction can be the conventional temperature of such reaction in the art, and preferably, the temperature is 60-70°C in the present application.

In the preparation method, the reaction progress can be monitored by the conventional monitoring method in the art (such as TLC, HPLC or NMR), and generally, the reaction endpoint is when the compound shown in formula II disappears or no longer reacts. The reaction has a time of 6-12 hours, for example, 10 h.

The present application also provides an application of the carboxylic acid compound shown in formula I or the salt thereof as an extractant.

In the application, the extractant is one or a mixture (for example, two or more) of the carboxylic acid compounds shown in formula I, for example, any one or a mixture (for example, two or more) of the following compounds:

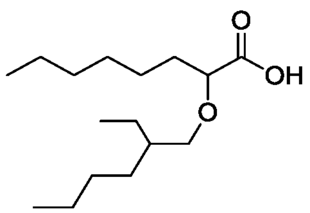,
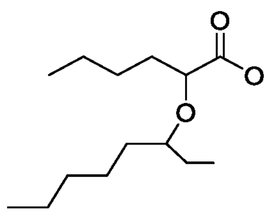,
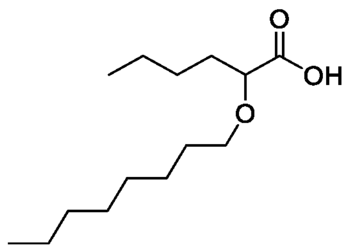,

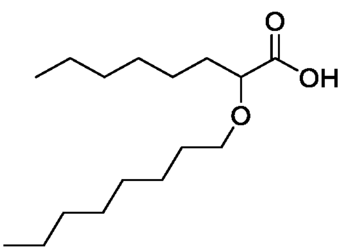,
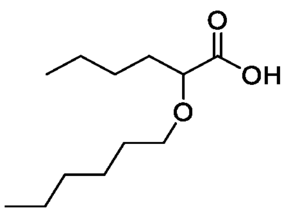, and
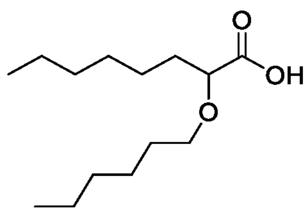.

In the application, the carboxylic acid compound shown in formula I or the salt thereof is used as an extractant for extracting and separating an metal ion. Preferably, the metal ion is one or a mixture (for example, two or more) of $Ni^{2+}$, $Co^{2+}$ and $Mn^{2+}$, the metal ion can further include one or a mixture (for example, two or more) of $Fe^{3+}$, $Al^{3+}$, $Cu^{2+}$, $Zn^{2+}$, $Cd^{2+}$ and $Ca^{2+}$, and the metal ion can further include $Mg^{2+}$, $Li^{+}$ or other ions. For example, the metal ion is a combination of at least one of $Ni^{2+}$, $Co^{2+}$ and $Mn^{2+}$ with at least one of $Fe^{3+}$, $Al^{3+}$, $Cu^{2+}$, $Zn^{2+}$, $Cd^{2+}$, $Ca^{2+}$, $Mg^{2+}$ and

$Li^{+}$. For example, the metal ion is a mixture of $Ni^{2+}$, $Co^{2+}$, $Mn^{2+}$, $Fe^{3+}$, $Al^{3+}$, $Cu^{2+}$, $Zn^{2+}$, $Cd^{2+}$, $Ca^{2+}$, $Mg^{2+}$ and $Li^{+}$. Preferably, the metal ions can be those from waste lithium-ion battery positive electrode materials, nickel laterite ore or nickel-cobalt-containing waste residue. Therefore, in a preferred solution of the present application, the carboxylic acid compound shown in formula I or the salt thereof is used as an extractant for extracting and separating the metal ion from waste lithium-ion battery positive electrode materials, nickel laterite ore or nickel-cobalt-containing waste residue.

The present application provides an extraction composition, which includes an extractant and a diluent, in which the extractant includes the carboxylic acid compound shown in formula I and/or the salt of the carboxylic acid compound shown in formula I.

In the extraction composition, preferably, the carboxylic acid compound shown in formula I and the salt of the carboxylic acid compound shown in formula I have a molar ratio of (0.4-9):1 (for example, 1:1).

In the extraction composition, preferably, the extractant includes the carboxylic acid compound shown in formula I and the salt of the carboxylic acid compound shown in formula I, and the carboxylic acid compound shown in formula I and the salt of the carboxylic acid compound shown in formula I have a molar ratio of (0.4-9): 1.

In the extraction composition, the diluent can be the diluent commonly used in the art, preferably, the diluent is one or a mixture (for example, two or more) of solvent oil (for example, 200 # solvent oil or 260 # solvent oil), kerosene, Escaid 110, hexane, heptane and dodecane (for example, n-dodecane); more preferably, the diluent is one or a mixture (for example, two or more) of solvent oil (for example, 260 # solvent oil), dodecane (for example, n-dodecane) and Escaid 110.

In the extraction composition, an amount of the diluent is not particularly limited as long as the extraction and back extraction performance of the extraction composition are not affected. Preferably, the extractant and the diluent have a molar-volume ratio of 0.1-1.5 mol/L, preferably 0.16-0.85 mol/L, for example, 0.16 mol/L, 0.33 mol/L or 0.6 mol/L.

The present application provides an extraction method, which includes the following step: extracting an aqueous phase containing a metal ion with an organic phase containing an extractant to obtain an organic phase containing a metal ion;

in the organic phase containing an extractant, the extractant includes the carboxylic acid compound shown in formula I and/or the salt of the carboxylic acid compound shown in formula I;

in the aqueous phase containing a metal ion, the metal ion includes one or a mixture (for example, two or more) of $Ni^{2+}$, $Co^{2+}$, $Mn^{2+}$, $Fe^{3+}$, $Al^{3+}$, $Cu^{2+}$, $Zn^{2+}$, $Cd^{2+}$ and $Ca^{2+}$.

In the aqueous phase containing a metal ion, the metal ion can further include other ions such as $Mg^{2+}$ and $Li^{+}$. Preferably, the metal ion can be those from waste lithium-ion battery positive electrode materials, nickel laterite ore or nickel-cobalt-containing waste residue. Preferably, the metal ion is a combination of at least one of $Ni^{2+}$, $Co^{2+}$ and $Mn^{2+}$ with at least one of $Fe^{3+}$, $Al^{3+}$, $Cu^{2+}$, $Zn^{2+}$, $Cd^{2+}$, $Ca^{2+}$, $Mg^{2+}$ and $Li^{+}$. For example, the metal ion is a mixture of $Ni^{2+}$, $Co^{2+}$, $Mn^{2+}$, $Fe^{3+}$, $Al^{3+}$, $Cu^{2+}$, $Zn^{2+}$, $Cd^{2+}$, $Ca^{2+}$, $Mg^{2+}$ and $Li^{+}$.

In the organic phase containing an extractant, preferably, the carboxylic acid compound shown in formula I and the salt of the carboxylic acid compound shown in formula I have a molar ratio of (0.4-9): 1 (for example, 1: 1).

In the organic phase containing an extractant, preferably, the extractant includes the carboxylic acid compound shown in formula I and the salt of the carboxylic acid compound shown in formula I, and the carboxylic acid compound shown in formula I and the salt of the carboxylic acid compound shown in formula I have a molar ratio of (0.4-9): 1.

In the extraction method, preferably, the organic phase containing an extractant further includes a diluent. The diluent can be the diluent commonly used in the art. Preferably, the diluent is one or a mixture (for example, two or more) of solvent oil (for example, 200 # solvent oil or 260 # solvent oil), kerosene, Escaid 110, hexane, heptane and dodecane (for example, n-dodecane); more preferably, the diluent is one or a mixture (for example, two or more) of solvent oil (for example, 260 # solvent oil), dodecane (for example, n-dodecane) and Escaid 110. An amount of the diluent is not particularly limited as long as the extraction and back extraction performance of the organic phase containing an extractant are not affected. Preferably, the organic phase containing an extractant and the diluent have a molar-volume ratio of 0.1-1.5 mol/L, preferably 0.16-0.85 mol/L, for example, 0.16 mol/L, 0.33 mol/L or 0.6 mol/L.

In the extraction method, a volume ratio of the organic phase containing an extractant to the aqueous phase containing a metal ion can be the conventional ratio for extraction in the art; preferably, the organic phase containing an extractant and the aqueous phase containing a metal ion have a volume ratio of 1:(1-10), more preferably 1:(1-5), for example, 1:1, 1:2 or 1:4.

In the extraction method, preferably, mass transfer is realized by shaking.

In the extraction method, preferably, a temperature of the extraction can be the temperature conventionally used in the art, preferably 10-50°C, and more preferably 25-40°C. A time of the extraction can be the conventional time in the art, preferably 5-60 minutes, for example, 15 minutes or 30 minutes.

The present application provides a back extraction method, which comprises the following step: mixing the

organic phase containing a metal ion obtained from the extraction method mentioned above with an acid aqueous solution.

In the back extraction method, the metal ions loaded in the organic phase containing an metal ion are transferred into the aqueous phase to obtain a metal ion enriched aqueous phase and a regenerated organic phase.

In the back extraction method, a molar concentration of the acid aqueous solution can be the molar concentration commonly used in such back extraction in the art, preferably 0.5-5 mol/L, more preferably 1-3 mol/L, for example, 1 mol/L or 2 mol/L. The molar concentration refers to a ratio of the acid's amount of substance to a total volume of the acid aqueous solution.

In the back extraction method, the acid in the acid aqueous solution can be the conventional acid in the art, preferably an inorganic acid. The inorganic acid is preferably one or more of hydrochloric acid, sulfuric acid, phosphoric acid and nitric acid, more preferably sulfuric acid.

In the back extraction method, a volume ratio of the organic phase containing a metal ion to the acid aqueous solution can be the conventional ratio in the art, preferably (1-50):1, more preferably (10-20):1, for example, 10:1 or 15:1.

In the present application, the shaking is required for mass transfer to uniformly mixing the organic phase and the aqueous phase, which can be replaced by other conventional operations in the field, for example, stirring.

Without violating the common knowledge in the art, the above preferred conditions can be arbitrarily combined to obtain preferred examples of the present application.

The reagents and raw materials used in the present application are all commercially available.

The present application has the positive and progressive effects as follows:

(1) When applied to the extraction and separation of metal ions, the carboxylic acid compound of the present application has a high separation coefficient, low back extraction acidity, high loading rate (the saturation capacity for $Ni^{2+}$ is more than or equal to 16 g/L), and high back extraction rate (the first back extraction rate is more than 99%);

(2) The carboxylic acid compound of the present application has high stability and low water solubility (the oil content extracted from the extraction equilibrium system at pH 7.23 is less than or equal to 75 mg/L) as an extractant, which guarantees a stable extraction process and can reduce environmental pollution and cost;

(3) The carboxylic acid compound of the present application has low cost and promising application prospects, which can be used in various systems such as ternary battery recycling and battery-grade nickel sulfate preparation.

## BRIEF DESCRIPTION OF DRAWINGS

FIG. 1 shows extraction rate *E*%-pH curves of Compound BC196 for each ion.

## DETAILED DESCRIPTION

The present application is further illustrated by the embodiments, but the present application is not limited by the embodiments. The experimental methods without specific conditions in the embodiments are selected from the conventional methods and conditions, or the product specifications.

The information about experiments in the embodiments is as follows.

The organic phase refers to an organic phase containing an extractant and a diluent, in which the extractant includes the carboxylic acid compound shown in formula I and the salt of the carboxylic acid compound shown in formula I.

The aqueous phase refers to an aqueous phase containing metal ions, wherein the aqueous phase containing metal ions can be prepared by the conventional methods, which, for example, include the following steps: dissolving a certain amount of a salt in deionized water and diluting the solution to a required concentration.

The ratio (O:A) refers to a volume ratio of an organic phase to an aqueous phase.

The term "saponification" refers to the replacement of a hydrogen ion in the extractant by an alkali metal ion and/or $NH_4^+$ (the obtained alkali metal ion and/or $NH_4^+$ can be exchanged with metal ions to be extracted in the aqueous phase to achieve extraction); the saponification includes the step: mixing the organic phase with the base aqueous solution. Preferably, the base aqueous solution used in the saponification can be an aqueous solution of sodium hydroxide, an aqueous solution of potassium hydroxide or ammonia.

The saponification proportion refers to the proportion of the alkali metal and/or $NH_4^+$ to the original hydrogen ion, i.e.,

$$\eta=(V_{base}\times C_{base})/(V_{org}\times C_{org}) \qquad (1).$$

In equation (1), $V_{base}$ is the volume of the base aqueous solution added, $C_{base}$ is the concentration of the base

aqueous solution added, $V_{org}$ is the volume of the organic phase, and $C_{org}$ is the extractant concentration of the organic phase.

In the embodiments of the present application, the metal ion concentration of the aqueous phase is determined by the inductively coupled plasma optical emission spectroscopy (ICP-OES), and the metal ion concentration of the organic phase is calculated by the difference subtraction method.

Potentiometric titration for acid content, with reference to the literature: Yuan Chengye, Hu Shuisheng; Studies on Organophosphorus Compounds XVI. Substituent Constants σp for Long Chain Alkyl and Alkoxyl Groups and their Correlation with Group Connectivity [J]. Acta Chimica Sinica, 1986, 44, 590-596; potentiometric titrator: Metrohm 907 Titrando, Switzerland. The acid content is used to represent the extractant purity in the embodiments of the present application.

The distribution ratio *D* is a ratio of the metal ion content of the equilibrium organic phase to the metal ion content of the equilibrium aqueous phase after the first extraction (the metal ion concentration of the equilibrium aqueous phase is determined by the inductively coupled plasma optical emission spectroscopy (ICP-OES), and the metal ion concentration of the equilibrium organic phase is calculated by the difference subtraction method), i.e.,

$$D=C_{org}/C_{aq}=(C'_{aq}-C_{aq})/C_{aq} \qquad (2)$$

In equation (2), $C_{org}$ represents the metal ion concentration of the equilibrium organic phase after the first extraction; $C_{aq}$ represents the metal ion concentration of the equilibrium aqueous phase after the first extraction; $C'_{aq}$ represents the metal ion concentration of the aqueous phase before the first extraction.

The extraction rate *E* is the percentage of the amount of the extracted substance transferred to organic phase from aqueous phase during the extraction process against the total amount of the extracted substances in the original aqueous phase, i.e.,

$$E=100\%\times(C'_{aq}-C_{aq})/C'_{aq} \qquad (3)$$

In equation (3), $C_{aq}$ represents the metal ion concentration of the equilibrium aqueous phase after the first extraction; $C'_{aq}$ represents the metal ion concentration of the aqueous phase before the first extraction.

The separation coefficient β refers to a ratio of the distribution ratios of two substances to be separated in two phases under certain conditions, which is also known as extraction separation factor.

Raw materials for which no preparation method is provided in the embodiments are commercially available.

**Example 1**

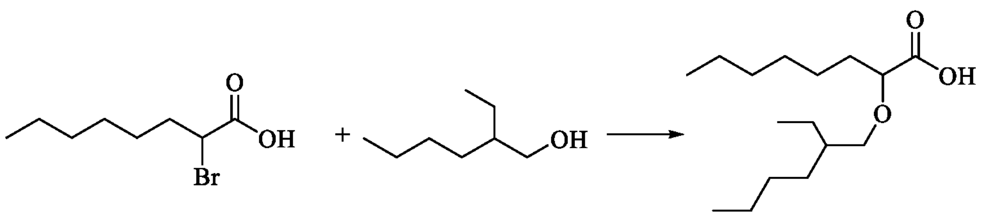

BC195

50 g of isooctanol, 225 mL of tetrahydrofuran (THF) and 8.8 g of sodium granules were added into a three-necked flask, and reacted at 60-70°C for 6 h; a large amount of white solid was generated and a small amount of sodium granules remained; at 60°C, 40 mL of THF solution containing 8 mol/L 2-bromooctanoic acid was added dropwise, and then reacted at 60°C for 4 h; after cooling, THF was removed by rotary evaporation, and then 200 mL of water and 200 mL of ethyl acetate (EA) were added into the concentrated solution, shaken and allowed to form layer separation, and the aqueous layer was collected; the aqueous layer was acidified with hydrochloric acid to a pH of about 1 and extracted with ethyl acetate, and then the organic phase was washed with water twice and dried by rotary evaporation, so as to obtain 65 g of light yellow product, i.e., Compound BC195. $^1H$ NMR (400 MHz, $CDCl_3$) δ 4.1 (1H), 3.52 (1H), 3.35 (1H), 1.82 (2H), 1.54 (3H), 1.20-1.31 (14H), 0.91 (6H), 0.87 (3H); $^{13}C$ NMR (101 MHz, $CDCl_3$) δ 171 (s), 79 (s), 72 (s), 36(s), 32 (s), 29 (s), 26-28 (m), 22-23 (m), 14 (s), 11 (s); MS $[M-H]^-$: 271.

**Example 2**

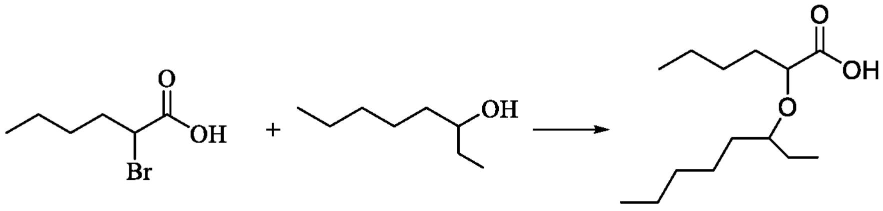

**BC196**

28.6 g of isooctanol, 200 mL of tetrahydrofuran (THF) and 8.8 g of 60% sodium hydride (dispersed in mineral oil) were added into a three-necked flask, and reacted at 60-70°C for 6 h; a large amount of white solid was generated and a small amount of sodium granules remained; at 60°C, 20 mL of THF solution containing 10 mol/L 2-bromohexanoic acid was added dropwise, and then reacted at 60°C for 4 h; after cooling, THF was removed by rotary evaporation, and then 200 mL of water and 200 mL of ethyl acetate (EA) were added into the concentrated solution, shaken and allowed to form layer separation, and the aqueous layer was collected; the aqueous layer was acidified with hydrochloric acid to a pH of about 1 and extracted with ethyl acetate, and then the organic phase was washed with water twice and dried by rotary evaporation, so as to obtain 38 g of light yellow product, i.e., Compound BC196. $^{1}H$ NMR (400 MHz, $CDCl_3$) $\delta$ 3.97 (1H), 3.41 (1H), 3.26 (1H), 1.70 (2H), 1.45 (3H), 1.05-1.24 (10H), 0.91 (9H); $^{13}C$ NMR (101 MHz, $CDCl_3$) $\delta$ 175 (s), 82 (s), 76 (s), 40 (s), 32 (s), 30 (s), 29 (s), 27 (s), 22-23 (m), 14 (s), 11 (s); MS$[M-H]^-$: 243.

**Example 3**

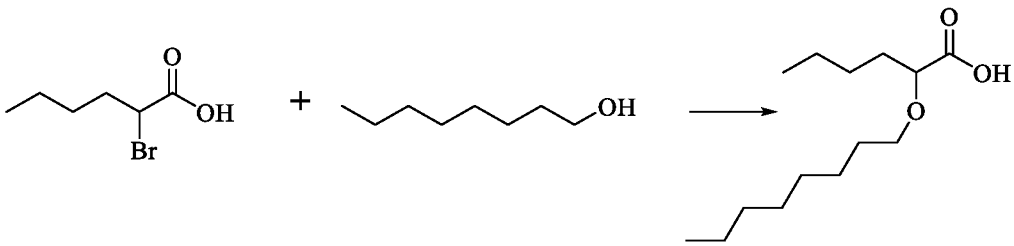

**BC191**

32 g of n-octanol, 200 mL of tetrahydrofuran (THF) and 5.7 g of sodium granules were added into a three-necked flask, and reacted at 60-70°C for 6 h; a large amount of white solid was generated and a small amount of sodium granules remained; at 60°C, 20 mL of THF solution containing 10 mol/L 2-bromohexanoic acid was added dropwise, and then reacted at 60°C for 4 h; after cooling, THF was removed by rotary evaporation, and then 200 mL of water and 200 mL of ethyl acetate (EA) were added into the concentrated solution, shaken and allowed to form layer separation, and the aqueous layer was collected; the aqueous layer was acidified with hydrochloric acid to a pH of about 1 and extracted with ethyl acetate, and then the organic phase was washed with water twice and dried by rotary evaporation, so as to obtain the target compound, i.e., Compound BC191.

Compound BC191 $^{1}H$ NMR (400 MHz, $CDCl_3$) $\delta$ 12.53 (1H), 4.01 (1H), 3.32 (2H), 1.65 (2H), 1.20-1.32 (16H), 0.89 (6H); $^{13}C$ NMR (101 MHz, $CDCl_3$) $\delta$ 173 (s), 81(s), 65 (s), 32-30 (m), 22-23 (m), 14 (s); MS$[M-H]^-$: 243.

**Example 4**

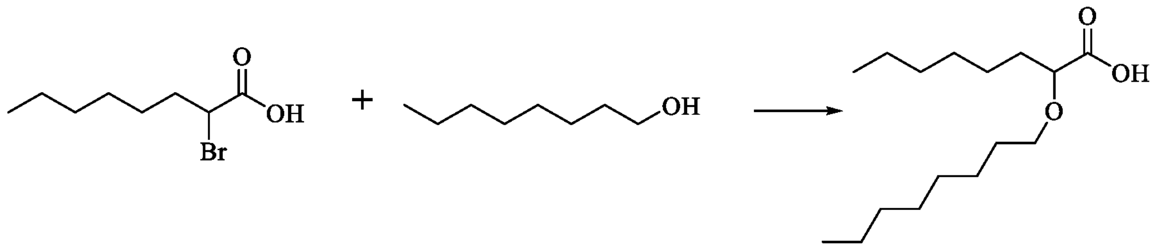

**BC192**

32 g of n-octanol, 200 mL of tetrahydrofuran (THF) and 5.7 g of sodium granules were added into a three-necked flask, and reacted at 60-70°C for 6 h; a large amount of white solid was generated and a small amount of sodium granules remained; at 60°C, 22 mL of THF solution containing 10 mol/L 2-bromooctanoic acid was added dropwise, and then reacted at 60°C for 4 h; after cooling, THF was removed by rotary evaporation, and then 200 mL of water and 200 mL of ethyl acetate (EA) were added into the concentrated solution, shaken and allowed to form layer separation, and the aqueous layer was collected; the aqueous layer was acidified with hydrochloric acid to a pH of about 1 and extracted with ethyl acetate, and then the organic phase was washed with water twice and dried by rotary evaporation, so as to obtain the target compound, i.e., Compound BC192.

Compound BC192 $^1$H NMR (400 MHz, $CDCl_3$) δ 11.54 (1H), 3.98 (1H), 3.30 (2H), 1.63 (2H), 1.42-1.44 (4H), 1.20-1.32 (16H), 0.89 (6H); MS$[M-H]^-$: 271.

**Example 5**

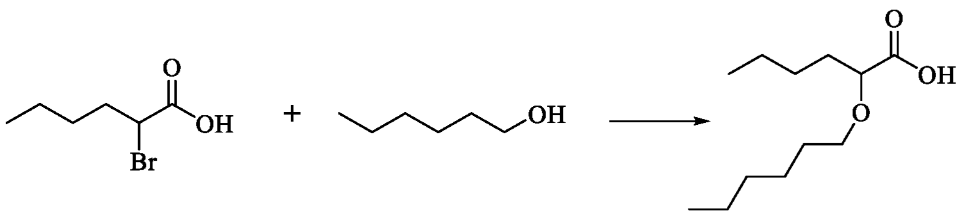

**BC193**

28 g of n-hexanol, 200 mL of tetrahydrofuran (THF) and 6.4 g of sodium granules were added into a three-necked flask, and reacted at 60-70°C for 6 h; a large amount of white solid was generated and a small amount of sodium granules remained; at 60°C, 20 mL of THF solution containing 10 mol/L 2-bromohexanoic acid was added dropwise, and then reacted at 60°C for 4 h; after cooling, THF was removed by rotary evaporation, and then 200 mL of water and 200 mL of ethyl acetate (EA) were added into the concentrated solution, shaken and allowed to form layer separation, and the aqueous layer was collected; the aqueous layer was acidified with hydrochloric acid to a pH of about 1 and extracted with ethyl acetate, and then the organic phase was washed with water twice and dried by rotary evaporation, so as to obtain the target compound, i.e., Compound BC193.

Compound BC193 $^1$H NMR (400 MHz, $CDCl_3$) δ 12.34 (1H), 3.89 (1H), 3.29 (2H), 1.61 (2H), 1.20-1.32 (10H), 0.89 (6H); MS$[M-H]^-$: 215.

**Example 6**

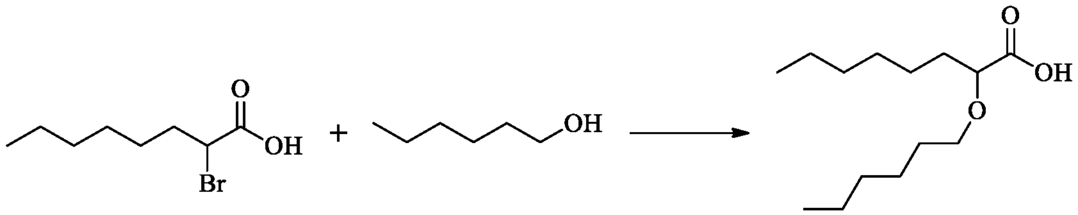

**BC194**

28 g of n-hexanol, 200 mL of tetrahydrofuran (THF) and 6.4 g of sodium granules were added into a three-necked flask, and reacted at 60-70°C for 6 h; a large amount of white solid was generated and a small amount of sodium granules remained; at 60°C, 22 mL of THF solution containing 10 mol/L 2-bromooctanoic acid was added dropwise, and then reacted at 60°C for 4 h; after cooling, THF was removed by rotary evaporation, and then 200 mL of water and 200 mL of ethyl acetate (EA) were added into the concentrated solution, shaken and allowed to form layer separation, and the aqueous layer was collected; the aqueous layer was acidified with hydrochloric acid to a pH of about 1 and extracted with ethyl acetate, and then the organic phase was washed with water twice and dried by rotary evaporation, so as to obtain the target compound, i.e., Compound BC194.

Compound BC194 $^1H$ NMR (400 MHz, $CDCl_3$) $\delta$ 12.86 (1H), 4.04 (1H), 3.37 (2H), 1.67 (2H), 1.42-1.44 (8H), 1.20-1.32 (8H), 0.89 (6H); MS$[M-H]^-$: 215.

**Performance Example 1** Extraction performance of Compound BC196

Compound BC196 has a structure:

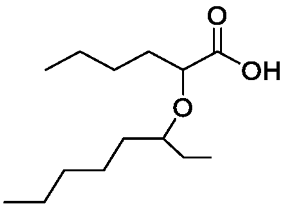

(an acid content is 98%) (the acid content refers to the extractant purity).

Compound BC196 was dissolved in a diluent, 260 # solvent oil, and prepared as a 0.6 mol/L organic phase, and a mixed sulfate solution was prepared as an aqueous phase, which contained 0.02 mol/L $Cu^{2+}$, $Zn^{2+}$, $Fe^{3+}$, $Al^{3+}$, $Cd^{2+}$, $Ni^{2+}$, $Co^{2+}$, $Mn^{2+}$, $Ca^{2+}$, $Mg^{2+}$ and $Li^+$. The organic phase was firstly saponified by 11.9 mol/L sodium hydroxide aqueous solution with a saponification rate of 0-70%, the initial pH of the aqueous phase remained unchanged at 2.08, and the aqueous phase was extracted by the organic phase with different saponification degree at a volume ratio of 1:1, the equilibrium time was 15 min and the temperature was 25°C.

After extraction, the extraction rate and equilibrium pH were plotted to obtain the extraction rate *E*%-pH curve of Compound BC196 for each ion. The results are shown in FIG. 1 and Table 1. The separation coefficient of Compound BC196 between various ions is shown in Table 2.

Table 1: Extraction rate *E%* of Compound BC196 for each ion

| Equilibrium pH | Extraction Rate E% | | | | | | | | | | |
|---|---|---|---|---|---|---|---|---|---|---|---|
| | $Fe^{3+}$ | $Cu^{2+}$ | $Ca^{2+}$ | $Al^{3+}$ | $Cd^{2+}$ | $Zn^{2+}$ | $Ni^{2+}$ | $Co^{2+}$ | $Mn^{2+}$ | $Mg^{2+}$ | $Li^+$ |
| 4.5 | 90.2 | 95.0 | 71.5 | 26.3 | 76.0 | 62.0 | 42.5 | 34.5 | 27.5 | 1.6 | 2.3 |

Table 2: Separation Coefficient of Compound BC196 between various ions

| | Separation Coefficient $\beta$ | | | | | | | | |
|---|---|---|---|---|---|---|---|---|---|
| | $Cu^{2+}$ | $Ca^{2+}$ | $Al^{3+}$ | $Cd^{2+}$ | $Zn^{2+}$ | $Ni^{2+}$ | $Co^{2+}$ | $Mn^{2+}$ | $Mg^{2+}$ |
| $Ca^{2+}$ | 7.57 | | | | | | | | |

(continued)

| | Separation Coefficient $\beta$ | | | | | | | | |
|---|---|---|---|---|---|---|---|---|---|
| | $Cu^{2+}$ | $Ca^{2+}$ | $Al^{3+}$ | $Cd^{2+}$ | $Zn^{2+}$ | $Ni^{2+}$ | $Co^{2+}$ | $Mn^{2+}$ | $Mg^{2+}$ |
| $Al^{3+}$ | 53.22 | 7.03 | | | | | | | |
| $Cd^{2+}$ | 6.00 | 0.79 | 0.11 | | | | | | |
| $Zn^{2+}$ | 11.64 | 1.54 | 0.22 | 1.94 | | | | | |
| $Ni^{2+}$ | 25.71 | 3.40 | 0.48 | 4.29 | 2.21 | | | | |
| $Co^{2+}$ | 36.05 | 4.76 | 0.68 | 6.01 | 3.10 | 1.40 | | | |
| $Mn^{2+}$ | 50.13 | 6.62 | 0.94 | 8.36 | 4.31 | 1.95 | 1.39 | | |
| $Mg^{2+}$ | 1165.64 | 153.93 | 21.90 | 194.29 | 100.12 | 45.34 | 32.33 | 23.25 | |
| $Li^{+}$ | 808.51 | 106.77 | 15.19 | 134.77 | 69.447 | 31.45 | 22.43 | 16.13 | 0.69 |

It can be seen from FIG. 1 and Table 1 that the extraction sequence of Compound BC196 goes: $Fe^{3+}$, $Cu^{2+}$, $Ca^{2+}$, $Al^{3+}$, $Cd^{2+}$, $Zn^{2+}$, $Ni^{2+}$, $Co^{2+}$, $Mn^{2+}$, $Mg^{2+}$ and $Li^{+}$; when the equilibrium pH value is 4.5, the extraction rate of Compound BC196 for Zn is about 65%, the extraction rate for Ni, Co and Mn is 25-45%, and Mg can hardly be extracted. It can be seen from Table 2 that the separation coefficients of Zn for Ni, Co, Mn are 2.21, 3.10 and 4.31 respectively, and the separation coefficients of Ni, Co, Mn for Mg are 45.34, 32.33 and 23.25 respectively. It can be seen that Compound BC196 extracts nickel, cobalt and manganese before magnesium ions, and nickel, cobalt and manganese have high separation degree from impurity metal ions such as magnesium and zinc. The above results show that, compared with the reported extractants P204, P507 and C272, Compound BC196 has better ion selectivity, which can synchronously recycle nickel, cobalt and manganese, and has feasible application value in the recovery of positive electrode materials of lithium-ion batteries.

**Performance Comparative Example** 1

This comparative example differs from Performance Example 1 in that Compound BC196 was replaced by Extractant CA12 (commercially available, with an acid content of 98%). The results are shown in Table 3.

Table 3: Separation coefficients of Compounds BC196 and CA12 for each ion

| System | pH | Metal ion | | | |
|---|---|---|---|---|---|
| | | $\beta_{Ni/Co}$ | $\beta_{Ni/Mn}$ | $\beta_{Ni/Mg}$ | $\beta_{Ni/Zn}$ |
| Compound BC196 | 4.5 | 1.40 | 1.95 | 45.34 | 2.21 |
| CA12 | 4.5 | 1.08 | 1.41 | 31.98 | 1.66 |

As can be seen from Table 3 that the separation coefficients of Compound BC196 for each ion are higher by about 20-30% compared with CA12 under the same test condition. Under the pH condition of about 4.5, the separation coefficients of Compound BC196 for Ni/Mg and Ni/Zn are 45.34 and 2.21, respectively, while the separation coefficients of CA12 for Ni/Mg and Ni/Zn are 31.98 and 1.66, respectively, which indicates that Compound BC196 has better ion separation performance than CA12.

**Performance Example 2** Back extraction performance of Compound BC196 loaded with metal ions

Compound BC196 was dissolved in dodecane and prepared as a 0.33 mol/L organic phase, and the aqueous phase used a 0.02 mol/L $Ni^{2+}$ sulfate solution to be a feed solution. The organic phase was saponified with 9 mol/L ammonia, the saponification proportion was 50%, the saponified organic phase extracted the feed solution with a phase ratio of 1:4, the equilibrium time was 15 min and the temperature was 25°C. The organic phase loaded with Ni was obtained, which had a Ni content of 0.08 mol/L.

The organic phase loaded with Ni was back-extracted with 1 mol/L sulfuric acid aqueous solution, and during the back extraction, the phase ratio was 10:1, and the back extraction rate was more than 99%.

However, the P507 organic phase loaded with Ni is generally back-extracted with 2 mol/L sulfuric acid, and the

first back extraction rate is about 85%. The above results show that when the carboxylic acid compound of the present application is applied to the extraction of metal ions, a higher back extraction rate can be obtained on the premise of lower back extraction acidity.

**Performance Example 3** Saturation capacity of Compound BC196 for $Ni^{2+}$

Test Method: Compound BC196 was dissolved in dodecane and prepared as a 0.6 mol/L organic phase. A 50 g/L $NiSO_4$ aqueous solution was prepared as an aqueous phase.

10 mL of the organic phase was added into a 50 mL separatory funnel, and saponified to a proportion of 60% with 10 mol/L NaOH aqueous solution, and with no need to wait the saponified organic phase to separate layers, 10 mL of aqueous phase was added directly, shaken and mixed for 15 min; the aqueous phase was separated, and then a fresh 50 g/L $NiSO_4$ aqueous phase (10 mL) was added, shaken and mixed for 15 min; the above operation was repeatedly carried out until the ion concentration in the aqueous phase did not change, and then the metal concentration of the organic phase was the saturation capacity of the extractant. The organic phase was back-extracted and the saturation capacity of Compound BC196 for $Ni^{2+}$ was obtained to be 16 g/L.

**Performance Example 4** Back extraction performance of Compound BC195 loaded with metal ions

Compound BC195 has a structure:

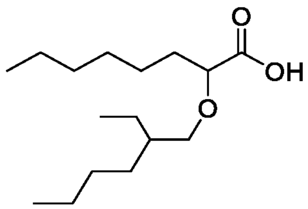

(an acid content is 95%).

Compound BC195 was dissolved in Escaid 110 and prepared as a 0.16 mol/L organic phase, and a 0.02 mol/L $Ni^{2+}$ sulfate solution was prepared as a feed liquid. The organic phase was saponified to a proportion of 50% with 10 mol/L NaOH aqueous solution, and the saponified organic phase extracted the feed solution with a phase ratio of 1:2, the equilibrium time was 15 min and the temperature was 25°C. The organic phase loaded with Ni was obtained, which had a Ni content of 0.04 mol/L.

The organic phase loaded with Ni was back-extracted with 1 mol/L sulfuric acid aqueous solution, and during the back extraction, the phase ratio was 15:1, and the back extraction rate was more than 99%.

However, the P507 organic phase loaded with Ni is generally back-extracted with 2 mol/L sulfuric acid, and the first back extraction rate is about 85%. The above results show that when the carboxylic acid compound of the present application is applied to the extraction of metal ions, a higher back extraction rate can be obtained on the premise of lower back extraction acidity.

**Performance Example 5** Solubility test of Extractant BC199 and Extractant CA12 in extraction systems

Extractant BC199 is obtained by mixing the following compounds with a molar ratio of 1:1:1:1:

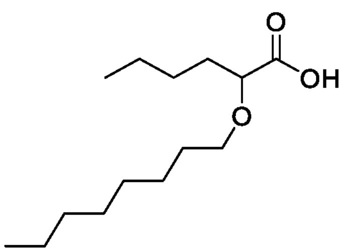 , 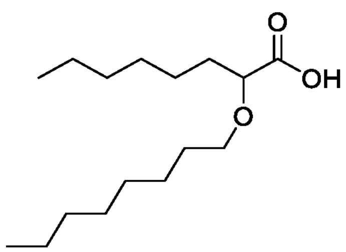 , 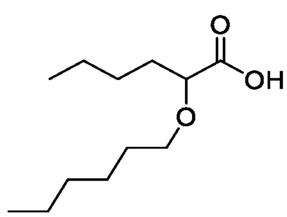 ,

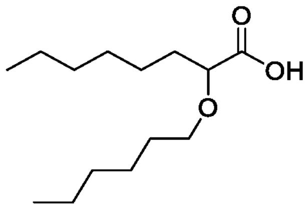

(an acid content is 99%).

Extraction: Extractant BC199 and diluent Escaid 110 were prepared to a 0.6 mol/L solution as an organic phase, each compound has a concentration of 0.15 mol/L in the organic phase, and an aqueous phase was a 0.2 mol/L $NiSO_4$ aqueous solution; 100 mL of the organic phase was added into a 250 mL separatory funnel, and 10 mol/L sodium hydroxide aqueous solution was added for saponification to a proportion of 24%, 100 mL of the aqueous phase was added, extraction equilibrium was carried out for 30 min, and the temperature was 25°C.

Oil content test: 50 mL of the aqueous phase reaching equilibrium was added into a 100 mL separatory funnel, and then added with a proper amount of HCl to adjust the pH value of the aqueous phase less than or equal to 2. The 25 mL of tetrafluoroethylene was accurately transferred into the separatory funnel with a pipette, shaken for 10 min and then allowed to stand. The tetrachloroethylene in the lower part of the separatory funnel was discharged into a conical flask, then anhydrous sodium sulfate was added into the conical flask to about 1 g/L and shaken, and the sodium sulfate should not agglomerate to ensure that the water in tetrachloroethylene was removed completely. With tetrachloroethylene as a blank group, the oil content in the sample was determined by infrared oil meter.

**Performance Comparative Example 2**

This comparative example differs from Performance Example 5 in that Compound BC199 was replaced by Extractant CA12 (commercially available, with an acid content of 98%). The solubility of Extractant CA12 in the extraction system was tested.

The test results of Performance Example 5 and Performance Comparative Example 2 are shown in Table 4.

Table 4: Solubility of Extractant BC199 and CA12 in extraction systems

| | CA12 | Extractant BC199 | Blank Diluent |
|---|---|---|---|
| Equilibrium pH of the System | 8.09 | 8.20 | - |
| Organic Compound Content mg/L | 6000 | 120 | 45 |

Through the above tests, it can be seen that the oil content extracted after the blank diluent (with no extractant added, and other operations were the same as Performance Example 5) reached equilibrium with the water phase is 45 mg/L, the oil content extracted after Extractant BC199 reached extraction equilibrium at pH 8.20 is about 120 mg/L, and the oil content extracted after CA12 reached extraction equilibrium at pH 8.09 is about 6000 mg/L. The results show that CA12 has a large dissolution loss in the extraction system, which causes an unstable process operation, and Extractant BC199 solves the problem of large extractant solubility in aqueous phase when used for extraction and separation of metal ions, which greatly reduces the process cost and ensures stable process operation.

**Performance Example 6**

Compound BC195 and diluent Escaid 110 were prepared into a 0.62 mol/L solution, and an aqueous phase was a magnesium-enriched nickel chloride solution containing 1.33 g/L Ni and 4 g/L Mg; 100 mL of the organic phase was added in a 250 mL separatory funnel, 10 mol/L sodium hydroxide aqueous solution was added for saponification to a saponification proportion of 24%, and after the saponification, 100 mL of the aqueous phase was added, extraction equilibrium was carried out for 30 min, and the temperature was 25°C.

Oil content test: the aqueous phase was separated out and added with $H_2SO_4$, and the $[H^+]$ concentration of the aqueous phase solution was about 1 mol/L. The CHzClz was used for extraction (30 mL×3), and the $CH_2Cl_2$ layer was collected, dried with 1 g anhydrous $Na_2SO_4$ to remove the water in $CH_2Cl_2$, and filtered; the filtrate was subjected to rotary evaporation, and then the residue was dried with an oil pump for 30 min. The oil content which CHzClz extracted out in the system was obtained by weighing the flask before and after the rotary evaporation.

**Performance Comparative Example 3**

This comparative example differs from Performance Example 6 in that Compound BC195 was replaced by Extractant CA12 (commercially available, with an acid content of 98%).

The test results of Performance Example 6 and Performance Comparative Example 3 are shown in Table 5.

Table 5: Solubility of Compound BC195 and Compound CA12 in extraction systems

| | CA12 | Compound BC195 | Blank Diluent |
|---|---|---|---|
| Equilibrium pH | 7.15 | 7.23 | - |
| Organic Compound Content mg/L | 4180 | 75 | 45 |

Through the above tests, it can be seen that the oil content extracted after the blank diluent (with no extractant added, and other operations were the same as Performance Example 6) reached equilibrium with the water phase is 45 mg/L, the oil content extracted after Compound BC195 reached extraction equilibrium at pH 7.2 is about 75 mg/L, and the oil content for CA12 is about 4180 mg/L. CA12 has a large dissolution loss in the extraction system. Compound BC195 solves the problem of large extractant solubility in aqueous phase when used for extraction and separation of metal ions, which ensures stable process operation and reduces the process cost.

**Claims**

1. A carboxylic acid compound shown in formula I or a salt thereof:

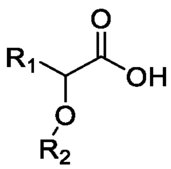

I ,

wherein $R_1$ and $R_2$ are independently selected from $C_3$-$C_{12}$ linear or branched alkyl.

2. The carboxylic acid compound shown in formula I or the salt thereof according to claim 1,

wherein $R_1$ is $C_4$-$C_9$ linear or branched alkyl;
and/or $R_2$ is $C_3$-$C_{10}$ linear or branched alkyl;
and/or the salt of the carboxylic acid compound shown in formula I is prepared by reacting the carboxylic acid compound shown in the formula I with a base according to a molar ratio of 1:1.

3. The carboxylic acid compound shown in formula I or the salt thereof according to claim 2,

wherein $R_1$ is $C_4$-$C_9$ linear alkyl, for example, n-butyl, n-pentyl, n-hexyl or n-octyl;
and/or $R_2$ is $C_6$-$C_8$ linear or branched alkyl; for example, $R_2$ is n-hexyl, n-octyl or isooctyl (for example,

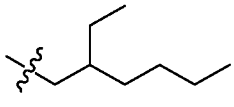 or 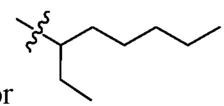 );

and/or a total carbon number n of $R_1$ and $R_2$ is 10-20, and for example, n is 12, 14 or 16.

4. The carboxylic acid compound shown in formula I or the salt thereof according to at least one of claims 1 to 3, wherein the carboxylic acid compound shown in formula I is selected from any one of the following compounds:

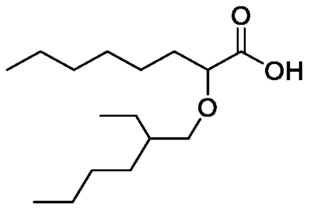, 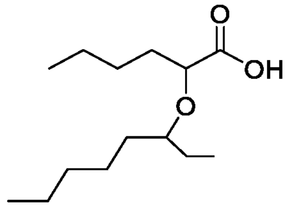, 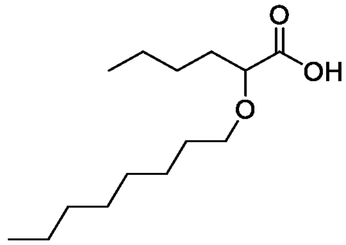,

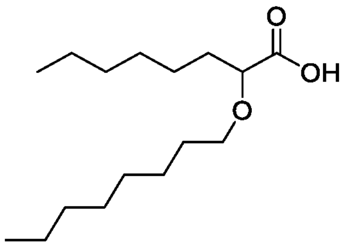, 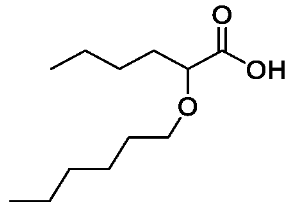, or 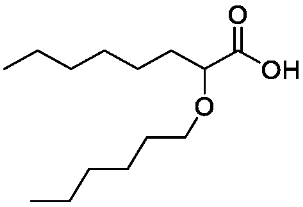.

5. A preparation method for the carboxylic acid compound shown in formula I according to at least one of claims 1 to 4, comprising reacting a compound shown in formula II with a compound shown in formula III in a solvent under the action of a base;

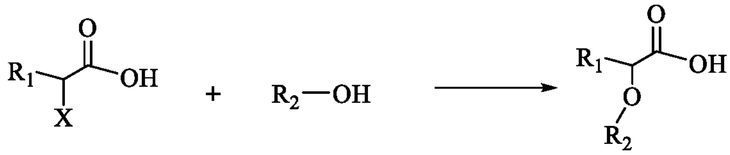

II III I

wherein X is halogen, and $R_1$ and $R_2$ are as defined by at least one of claims 1 to 4.

6. The preparation method for the carboxylic acid compound shown in formula I according to claim 5, wherein the halogen is fluorine, chlorine, bromine or iodine, for example, chlorine or bromine, or bromine;

and/or the solvent is an ether solvent, for example, tetrahydrofuran;
and/or the solvent and the compound shown in formula III have a volume-mass ratio of 1-10 mL/g, for example, 5.3 mL/g, 6.25 mL/g, 7.0 mL/g, 7.1 mL/g or 7.7 mL/g;
and/or the base is an alkali metal or an alkali metal hydride, for example, sodium or sodium hydride;
and/or the base and the compound shown in formula II have a molar ratio of (1-1.5):1, for example, 1.1:1, 1.2:1 or 1.35:1;
and/or the compound shown in formula II and the compound shown in formula III have a molar ratio of 1:(1-1.5), for example, 1: 1.1 or 1:1.2;
and/or the reaction has a temperature of 60-70°C;
and/or the reaction has a time of 6-12 hours, for example, 10 h.

7. An application of the carboxylic acid compound shown in formula I or the salt thereof according to at least one of claims 1 to 4 as an extractant.

8. The application of the carboxylic acid compound shown in formula I or the salt thereof as an extractant according to claim 7, wherein the extractant is one or a mixture of the carboxylic acid compounds shown in formula I;
and/or the carboxylic acid compound shown in formula I or the salt thereof is used as an extractant for extracting and separating an metal ion; preferably, the metal ion is one or a mixture of $Ni^{2+}$, $Co^{2+}$ and $Mn^{2+}$, the metal ion can further comprise one or a mixture of $Fe^{3+}$, $Al^{3+}$, $Cu^{2+}$, $Zn^{2+}$, $Cd^{2+}$ and $Ca^{2+}$, and the metal ion can further comprise $Mg^{2+}$ and/or $Li^+$.

**9.** The application of the carboxylic acid compound shown in formula I or the salt thereof as an extractant according to claim 8, wherein the extractant is selected from any one or a mixture of the following compounds:

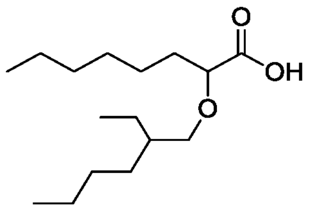, 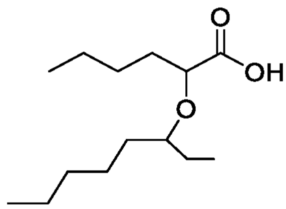, 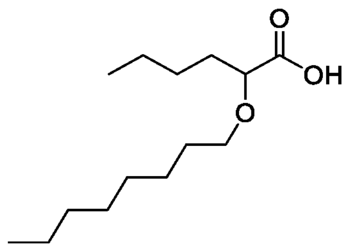,

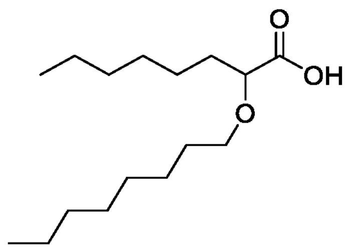, 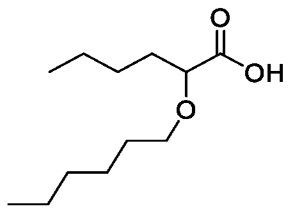, and 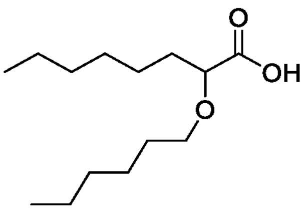;

and/or when the carboxylic acid compound shown in formula I or the salt thereof is used as an extractant for extracting and separating an metal ion, the metal ion is a combination of at least one of $Ni^{2+}$, $Co^{2+}$ and $Mn^{2+}$ with at least one of $Fe^{3+}$, $Al^{3+}$, $Cu^{2+}$, $Zn^{2+}$, $Cd^{2+}$, $Ca^{2+}$, $Mg^{2+}$ and $Li^{+}$; preferably, the metal ion is a mixture of $Ni^{2+}$, $Co^{2+}$, $Mn^{2+}$, $Fe^{3+}$, $Al^{3+}$, $Cu^{2+}$, $Zn^{2+}$, $Cd^{2+}$, $Ca^{2+}$, $Mg^{2+}$ and $Li^{+}$.

**10.** An extraction composition, comprising an extractant and a diluent, wherein the extractant comprises the carboxylic acid compound shown in formula I according to at least one of claims 1 to 4 and/or the salt of the carboxylic acid compound shown in formula I according to at least one of claims 1 to 4.

**11.** The extraction composition according to claim 10, wherein the carboxylic acid compound shown in formula I and the salt of the carboxylic acid compound shown in formula I have a molar ratio of (0.4-9):1, for example, 1:1; preferably, the extractant comprises the carboxylic acid compound shown in formula I and the salt of the carboxylic acid compound shown in formula I, and more preferably, the carboxylic acid compound shown in formula I and the salt of the carboxylic acid compound shown in formula I have a molar ratio of (0.4-9):1;

and/or the diluent is one or a mixture of solvent oil (for example, 200 # solvent oil or 260 # solvent oil), kerosene, Escaid 110, hexane, heptane and dodecane (for example, n-dodecane); preferably, the diluent is one or a mixture of solvent oil (for example, 260 # solvent oil), dodecane (for example, n-dodecane) and Escaid 110;
and/or the extractant and the diluent have a molar-volume ratio of 0.1-1.5 mol/L, preferably 0.16-0.85 mol/L, for example, 0.16 mol/L, 0.33 mol/L or 0.6 mol/L.

**12.** An extraction method, comprising extracting an aqueous phase containing a metal ion with an organic phase containing an extractant to obtain an organic phase containing a metal ion;

in the organic phase containing an extractant, the extractant comprises the carboxylic acid compound shown in formula I according to at least one of claims 1 to 4 and/or the salt of the carboxylic acid compound shown in formula I according to at least one of claims 1 to 4;
in the aqueous phase containing a metal ion, the metal ion comprises one or a mixture of $Ni^{2+}$, $Co^{2+}$, $Mn^{2+}$, $Fe^{3+}$, $Al^{3+}$, $Cu^{2+}$, $Zn^{2+}$, $Cd^{2+}$ and $Ca^{2+}$.

**13.** The extraction method according to claim 12, wherein the metal ion is a combination of at least one of $Ni^{2+}$, $Co^{2+}$ and $Mn^{2+}$ with at least one of $Fe^{3+}$, $Al^{3+}$, $Cu^{2+}$, $Zn^{2+}$, $Cd^{2+}$, $Ca^{2+}$, $Mg^{2+}$ and $Li^{+}$; preferably, the metal ion is a mixture of $Ni^{2+}$, $Co^{2+}$, $Mn^{2+}$, $Fe^{3+}$, $Al^{3+}$, $Cu^{2+}$, $Zn^{2+}$, $Cd^{2+}$, $Ca^{2+}$, $Mg^{2+}$ and $Li^{+}$;

and/or in the organic phase containing an extractant, the carboxylic acid compound shown in formula I and the

salt of the carboxylic acid compound shown in formula I have a molar ratio of (0.4-9):1, for example, 1:1; preferably, the extractant comprises the carboxylic acid compound shown in formula I and the salt of the carboxylic acid compound shown in formula I; more preferably, the carboxylic acid compound shown in formula I and the salt of the carboxylic acid compound shown in formula I have a molar ratio of (0.4-9):1;
and/or the organic phase containing an extractant further comprises a diluent, wherein the diluent is one or a mixture of solvent oil (for example, 200 # solvent oil or 260 # solvent oil), kerosene, Escaid 110, hexane, heptane and dodecane (for example, n-dodecane); preferably, the diluent is one or a mixture of solvent oil (for example, 260 # solvent oil), dodecane (for example, n-dodecane) and Escaid 110;
the extractant and the diluent have a molar-volume ratio of 0.1-1.5 mol/L, preferably 0.16-0.85 mol/L, for example, 0.16 mol/L, 0.33 mol/L or 0.6 mol/L;
and/or the organic phase containing an extractant and the aqueous phase containing a metal ion have a volume ratio of 1:(1-10), preferably 1:(1-5), for example, 1:1, 1:2 or 1:4;
and/or mass transfer is realized by shaking in the extraction method;
and/or the extraction has a temperature of 10-50°C, preferably 25-40°C;
and/or the extraction has a time of 5-60 minutes, for example, 15 minutes or 30 minutes.

**14.** A back extraction method, comprising mixing the organic phase containing a metal ion obtained from the extraction method according to claim 12 or 13 with an acid aqueous solution.

**15.** The back extraction method according to claim 14, wherein the acid aqueous solution has a molar concentration of 0.5-5 mol/L, preferably 1-3 mol/L, for example, 1 mol/L or 2 mol/L;

and/or an acid in the acid aqueous solution is an inorganic acid, and the inorganic acid is preferably one or more of hydrochloric acid, sulfuric acid, phosphoric acid and nitric acid, more preferably sulfuric acid;
and/or the organic phase containing a metal ion and the acid aqueous solution have a volume ratio of (1-50):1, more preferably (10-20):1, for example, 10:1 or 15:1.

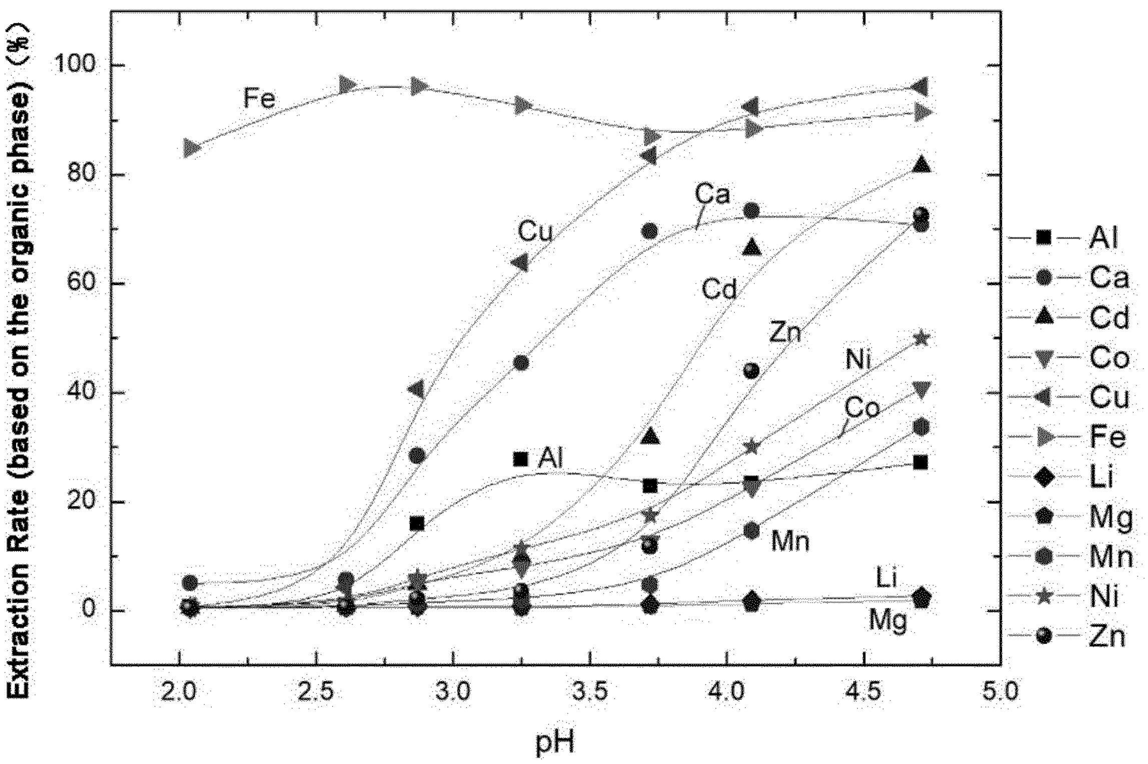
Extraction Rate (based on the organic phase) (%)
100
80
60
40
20
0
2.0
2.5
3.0
3.5
4.0
4.5
5.0
pH
Fe
Cu
Ca
Cd
Zn
Ni
Co
Al
Mn
Li
Mg
Al
Ca
Cd
Co
Cu
Fe
Li
Mg
Mn
Ni
Zn

FIG. 1

## INTERNATIONAL SEARCH REPORT

International application No.
**PCT/CN2020/123531**

**A. CLASSIFICATION OF SUBJECT MATTER**

C07C 59/125(2006.01)i; C07C 51/367(2006.01)i; C22B 3/12(2006.01)i; C22B 23/00(2006.01)i; C22B 47/00(2006.01)i

According to International Patent Classification (IPC) or to both national classification and IPC

**B. FIELDS SEARCHED**

Minimum documentation searched (classification system followed by classification symbols)

C07C59/-; C07C51/-; C22B

Documentation searched other than minimum documentation to the extent that such documents are included in the fields searched

Electronic data base consulted during the international search (name of data base and, where practicable, search terms used)

CNPAT, EPODOC, WPI, STN（REGISTRY, CAPLUS）: 萃取剂, 金属离子, 羧酸, 脂肪酸, 烷酸, 氧基, +oxy, acid, extractant, structural formula search

**C. DOCUMENTS CONSIDERED TO BE RELEVANT**

| Category* | Citation of document, with indication, where appropriate, of the relevant passages | Relevant to claim No. |
|---|---|---|
| PX | CN 111592459 A (BEIJING BOCUI CYCLE TECHNOLOGY CO., LTD.) 28 August 2020 (2020-08-28)<br>claims 1-15 | 1-15 |
| X | JP 08-3100 A (NEW JAPAN CHEMICAL CO., LTD.) 09 January 1996 (1996-01-09)<br>description, paragraphs 0006-0026, embodiments 1-2 | 1-6 |
| X | CN 1100408 A (ONO PHARMACEUTICAL CO., LTD.) 22 March 1995 (1995-03-22)<br>claims 12, 21, embodiments 7(34), 7(36)-7(38) | 1-3 |
| X | WO 0177086 A1 (DUPONT PHARMACEUTICALS COMPANY) 18 October 2001 (2001-10-18)<br>description, page 93, compound 10 | 1-2 |
| X | GUEST, Herbert H. et al. "Chlorination of Certain Long Chain Esters"<br>*Journal of the American Chemical Society,* Vol. 66, 01 December 1944 (1944-12-01), ISSN: 0002-7863,<br>pp. 2074-2075, specifically see p. 2075, right-hand column, paragraph 1 | 1-3, 5-6 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **03 March 2021** | **26 March 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2020/123531**

**C. DOCUMENTS CONSIDERED TO BE RELEVANT**

| Category* | Citation of document, with indication, where appropriate, of the relevant passages | Relevant to claim No. |
|---|---|---|
| X | 小玉新太郎 (KODAMA, Shintaro). "Rotatory Power of Alkylleucic Acids and Allied Compounds" *日本化学会志 (Journal of the Chemical Society of Japan),* Vol. 43, 31 December 1922 (1922-12-31), ISSN: 0369-4208, pages 704-734, specifically see pages 707, 710 | 1-3, 5-6 |
| X | STN. "RN 2303058-87-7 etc." *REGISTRY,* 08 April 2019 (2019-04-08), pp. 1-52 | 1-4 |
| A | CN 1084574 A (SHANGHAI INSTITUTE OF ORGANIC CHEMISTRY, CHINESE ACADEMY OF SCIENCES) 30 March 1994 (1994-03-30) description pages 1-2, embodiments 1-4, 8 | 1-15 |

Form PCT/ISA/210 (second sheet) (January 2015)

**INTERNATIONAL SEARCH REPORT**
**Information on patent family members**

International application No.
**PCT/CN2020/123531**

| Patent document cited in search report | | | Publication date (day/month/year) | Patent family member(s) | | | Publication date (day/month/year) |
|---|---|---|---|---|---|---|---|
| CN | 111592459 | A | 28 August 2020 | None | | | |
| JP | 08-3100 | A | 09 January 1996 | None | | | |
| CN | 1100408 | A | 22 March 1995 | CA | 2124784 | A1 | 02 December 1994 |
| | | | | JP | H09118644 | A | 06 May 1997 |
| | | | | JP | 2756756 | B2 | 25 May 1998 |
| | | | | JP | 2935110 | B2 | 16 August 1999 |
| | | | | DE | 69408373 | T2 | 16 July 1998 |
| | | | | US | 2005261371 | A1 | 24 November 2005 |
| | | | | KR | 100225299 | B1 | 15 October 1999 |
| | | | | ES | 2113574 | T3 | 01 May 1998 |
| | | | | JP | 3195581 | B2 | 06 August 2001 |
| | | | | US | 7176240 | B2 | 13 February 2007 |
| | | | | JP | H10204023 | A | 04 August 1998 |
| | | | | EP | 0632008 | B1 | 04 February 1998 |
| | | | | CA | 2124784 | C | 07 January 2003 |
| | | | | JP | H10324626 | A | 08 December 1998 |
| | | | | JP | 2826995 | B2 | 18 November 1998 |
| | | | | US | 2005267167 | A1 | 01 December 2005 |
| | | | | US | 2003096802 | A1 | 22 May 2003 |
| | | | | CN | 1083419 | C | 24 April 2002 |
| | | | | US | 2005267168 | A1 | 01 December 2005 |
| | | | | AT | 163006 | T | 15 February 1998 |
| | | | | GR | 3026076 | T3 | 29 May 1998 |
| | | | | CN | 1200703 | C | 11 May 2005 |
| | | | | US | 6201021 | B1 | 13 March 2001 |
| | | | | DE | 69408373 | D1 | 12 March 1998 |
| | | | | KR | 950000642 | A | 03 January 1995 |
| | | | | EP | 0632008 | A1 | 04 January 1995 |
| | | | | US | 7569609 | B2 | 04 August 2009 |
| | | | | CN | 1322524 | A | 21 November 2001 |
| | | | | DK | 0632008 | T3 | 23 September 1998 |
| | | | | TW | 248552 | B | 01 June 1995 |
| | | | | US | 7569608 | B2 | 04 August 2009 |
| | | | | JP | H07316092 | A | 05 December 1995 |
| | | | | KR | 100253725 | B1 | 15 April 2000 |
| WO | 0177086 | A1 | 18 October 2001 | US | 6900199 | B2 | 31 May 2005 |
| | | | | US | 7276495 | B2 | 02 October 2007 |
| | | | | US | 2005234039 | A1 | 20 October 2005 |
| | | | | US | 6632812 | B2 | 14 October 2003 |
| | | | | EP | 1289966 | A1 | 12 March 2003 |
| | | | | US | 7498324 | B2 | 03 March 2009 |
| | | | | US | 7390802 | B2 | 24 June 2008 |
| | | | | JP | 2004500419 | A | 08 January 2004 |
| | | | | US | 7655647 | B2 | 02 February 2010 |
| | | | | US | 2006211679 | A1 | 21 September 2006 |
| | | | | US | 2008221087 | A1 | 11 September 2008 |
| | | | | CA | 2404273 | A1 | 18 October 2001 |
| | | | | AU | 5700601 | A | 23 October 2001 |
| | | | | US | 2002025955 | A1 | 28 February 2002 |
| | | | | US | 2009170831 | A1 | 02 July 2009 |

Form PCT/ISA/210 (patent family annex) (January 2015)

**INTERNATIONAL SEARCH REPORT**
**Information on patent family members**

International application No.
**PCT/CN2020/123531**

| Patent document cited in search report | | | Publication date (day/month/year) | Patent family member(s) | | | Publication date (day/month/year) |
|---|---|---|---|---|---|---|---|
| | | | | US | 2004138201 | A1 | 15 July 2004 |
| | | | | US | 2007173491 | A1 | 26 July 2007 |
| CN | 1084574 | A | 30 March 1994 | CN | 1034818 | C | 07 May 1997 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202010599602
- CN 109449523 A

### Non-patent literature cited in the description

- **YUAN CHENGYE ; HU SHUISHENG.** Studies on Organophosphorus Compounds XVI. Substituent Constants σp for Long Chain Alkyl and Alkoxyl Groups and their Correlation with Group Connectivity [J]. *Acta Chimica Sinica,* 1986, vol. 44, 590-596